## Europäisches Patentamt

## European Patent Office

## Office européen des brevets

(19)

(11) Veröffentlichungsnummer: **0 137 500**
**B1**

(12) **EUROPÄISCHE PATENTSCHRIFT**

(45) Veröffentlichungstag der Patentschrift:
**14.09.88**

(51) Int. Cl.⁴: **A 61 B 5/04**

(21) Anmeldenummer: **84112197.3**

(22) Anmeldetag: **11.10.84**

(54) **Physiologische Messonde.**

(30) Priorität: **13.10.83 DE 3337188**
**21.09.84 DE 3434657**

(43) Veröffentlichungstag der Anmeldung:
**17.04.85 Patentblatt 85/16**

(45) Bekanntmachung des Hinweises auf die Patenterteilung:
**14.09.88 Patentblatt 88/37**

(84) Benannte Vertragsstaaten:
**AT BE CH DE FR GB IT LI LU NL SE**

(56) Entgegenhaltungen:
**DE - A - 2 619 471**
**GB - A - 1 467 344**
**US - A - 3 543 760**
**US - A - 3 750 650**
**US - A - 4 080 961**

**JOURNAL OF THE ACOUSTIC SOCIETY OF AMERICA,
Band 56, Nr. 2, August 1974, Seiten 708-711, New York,
US; A.C. COATS: "On electrocochleographic electrode
design"**

(73) Patentinhaber: **Morgenstern, Jürgen, Prof. Dr., Im
Heidewinkel 33, D-4000 Düsseldorf (DE)**

(72) Erfinder: **Morgenstern, Jürgen, Prof. Dr., Im
Heidewinkel 33, D-4000 Düsseldorf (DE)**

(74) Vertreter: **Plöger, Ulrich, Dipl.-Ing., Benrather
Schlossallee 89, D-4000 Düsseldorf-Benrath (DE)**

## Beschreibung

Die Erfindung bezieht sich auf eine physiologische Messsonde, insbesondere auf eine transzervikal mit Spitzen an der fetalen Kopfhaut fixierbare Vaginalelektrode für die Ableitung des fetalen EKG.

Eine derartige Messsonde ist nach der US-A-4 080 961 bekannt. Die den Kontakt mit der Kopfhaut herstellenden, schräg nach vorne geneigten Spitzen befinden sich dabei an den Enden von Federschenkeln, die sich in zwei voneinander getrennten Ebenen aneinander vorbeischieben lassen. Sie können sich dabei zu einem in seinen Abmessungen nicht definierten Spalt schliessen, indem eine Hülse über die Schenkel der Feder geschoben wird. Beim Zurückschieben der Hülse entspannt sich die Feder, ohne durch Anschläge oder dergl. hierbei begrenzt zu sein. Die zunächst aneinander vorbeischerenden Spitzen definieren mithin auch im entlasteten Zustand der Feder keinen vorgegeben, festen Abstand voneinander.

Derartige Messsonden sind ihrem grundsätzlichen Aufbau nach zur Ableitung des fetalen EKG während des gesamten Geburtsvorganges geeignet. Nach Edward H. Hon, Amer. J. Obstet. Gynec. Band 86, 1963, Seite 773, ist eine derartige Messsonde als Vaginalelektrode mit Kontaktspitzen ausgeführt, indem eine Wundklammer modifiziert und mittels einer Zange anwendbar gemacht wurde. Im Prinzip handelt es sich hierbei um eine Nadelelektrode. Sie setzt also ein verhältnismässig weites Eindringen der Spitze in die fetale Kopfhaut voraus. Vor Geburt des Kindes kann man eine derartige Klammer nur schwer wieder lösen. Darüber hinaus ist sie auch durch das Zu- und Aufbiegen für eine mehrfache Benutzung nicht geeignet, wie von H.W. Junge, 9, Geburtsh. u. Frauenheilk., 29.2, Seite 133, festgestellt wurde.

Weiterhin sind federnde, nadelförmige Elektroden, die mit ihren Kontaktspitzen teilweise aneinander vorbeigleiten und dadurch auch Gewebeschäden verursachen können, bekannt nach Edward H. Hon, Yale Journal of Biology and Medicine, Band 32, April 1960. Die Kontaktspitzen dringen durch Schliessen einer Klammer in die Kopfhaut ein, wobei von der Klammer eine Ableitung ausgeht. Die Gegenelektrode endigt an einer von der genannten Klammer isolierten Stelle frei, so dass sich ein Kontakt mit der Mutter ergibt. Die Klammer kann auch nach Charles A. Hunter, Kenneth G. Lansford, Suzanne B. Knoebel und Robert J. Braunlin, Obstetrics and Gynecology, Band 16, 1960, Seite 567 durch eine Feder ersetzt sein. In diesem Falle ist die Feder in einem Führungsrohr verschieblich angeordnet, aus welchem lediglich die Vorderabschnitte mit den abgewinkelten Kontaktspitzen hervorragen. Zum Schliessen der Klammer bzw. zum Schliessen der Feder muss man in jedem Falle zunächst noch zusätzliche Kräfte aufbringen, die unter Geburtsbedingungen die Konzentration des Arztes unnötig beanspruchen.

Demgegenüber liegt der Erfindung die Aufgabe zugrunde, eine physiologische Messsonde der einleitend bezeichneten Art dahingehend weiterzuentwickeln, dass sie einerseits eine wesentlich verbesserte Kontaktgabe ermöglicht, und dass andererseits das Hautgewebe bei der Applikation eine schonendere Behandlung erfährt. Vor allem soll die neue Messsonde auch die Möglichkeit der Anbringung weiterer Messelemente bieten, die mit der Applikationsfläche des Kopfes in Beziehung zu bringen sind, um den Geburtsvorgang nicht nur elektrokardiographisch, sondern auch durch sonstige Messungen überwachen zu können.

Diese Aufgabenstellung wird durch die Erfindung, wie sie in den Patentansprüchen gekennzeichnet ist, gelöst.

Danach liegt der Erfindung das Prinzip zugrunde, den Abstand der Spitzen voneinander durch eine in den Entspannungszustand zu überführende Feder in der Applikationsstellung auf ein vorgebbares Mass zu fixieren und ein gegebenenfalls einzusetzendes, weiteres Messorgan mit definierter Kraft an die Hautfläche anzupressen.

Bei einer ersten grundlegenden Ausführungsform der Erfindung wird von den weitergehenden Merkmalen der US-A-4 080 961 Gebrauch gemacht, wonach sich an die Messsonde ein Ableitkabel anschliesst und sie als Klammer ausgebildet ist, deren Klammerschenkeln mit den nach innen gerichteten Spitzen versehen sind, deren Abstand voneinander kleiner als der Abstand der Klammerschenkel ist, und an die sich eine Feder anschliesst, deren gespanntem Zustand die Öffnungslage der Klammerschenkel entspricht, und die sich bei Entlastung der Feder in Richtung aufeinander bewegen.

Bei dieser ersten, grundlegenden Ausführungsform sieht die Erfindung die Lösung der Aufgabenstellung durch die Vorschläge der Patentansprüche 1 bis 9 vor. Demnach brauchen die Spitzen nicht notwendigerweise in die Kopfhaut einzudringen, denn sie dienen lediglich dem Halten. Der eigentliche Kontakt bildet sich durch Anlage der Kopfhautfalte an die Klammerschenkel.

Der von den Klammerschenkeln nach Höhe und Breite vorgegebene Spalt besitzt dabei eine vorgebbare Weite, so dass der optimale Anpressdruck an die Kopfhaut gewährleistet wird. Letztere wird bei Anwendung von den Zähnen der geöffneten Klammerschenkel erfasst. Bei Entlasten der Feder schliessen sich die Klammerschenkel und ziehen dabei eine Hautfalte zwischen sich ein, die dann dem Druck der Schenkelflächen ausgesetzt ist. Die ausgedehnt flächige Anlage der Klammerschenkel bietet hervorragende Möglichkeiten, Geber von weiteren Messeinrichtungen mit den Klammerschenkeln zu verbinden, um sie gleichfalls auf die Kopfhaut zur Einwirkung zu bringen. Beispielsweise kann der eine Klammerschenkel eine schwache Lichtquelle und der andere Klammerschenkel eine Fotodiode aufnehmen, so dass Durchblutungsmessungen möglich sind. Die Klammerschenkel gestatten desweiteren die Anbringung von Elektroden für die Messung der Sauerstoffversorgung und die Anbringung von Beschleunigungsaufnehmern für die Überwachung der Geburtsmechanik. In allen Fällen ist die durch die erfindungsgemässe Messsonde ermöglichte, genau definierte Anlage der Klammerschenkelflächen an der zwischen ihnen eingezogenen Hautfalte von erheblichem Vorteil. Die Anwendung kann einerseits mittels einer Zange geschehen, indem die Zange an den Angriffsflächen der Feder anliegt und in gespanntem Zustand eingeführt wird. Bei Entlastung und Öffnung der Feder lässt sich die Zange zurückziehen. Umgekehrt ist eine Entfernung der Messsonde mittels der Zange gleichfalls leicht möglich. Darüber hinaus besteht aber auch die Möglichkeit, in an sich bekannter Weise einen verschiebbaren Kolben oder dergleichen zu verwenden, um die Feder aus gespanntem Zustand in den entspannten Zustand zu überführen. Die Feder ist zu diesem Zweck vorteilhaft in einer Hülse oder dergleichen angeordnet, aus der sie herausschiebbar ist.

Besonders zweckmässig ist die kreisringförmige Ausbildung der Feder, wobei dann die Spannung im ringförmigen Teil besteht, während die Klammerschenkel geradflächig verlaufen. Zwischen letzteren und der Feder können vorteilhaft gebogene Verbindungsabschnitte bestehen, die sich entlang einer in Richtung der Breite des Spaltes verlaufenden Linie berühren. Auf diese Weise wird ein Abrollpunkt geschaffen, so dass sich beim Zusammendrücken des kreisringförmigen Federteils die Klammerschenkel öffnen. In diesem Zustand werden die Zähne auf die zu ergreifende Haut aufgesetzt, woraufhin die Feder entspannt werden kann, was dann zu der bereits beschriebenen Aufnahme einer Hautfalte zwischen den Klammerschenkeln führt. Soweit die Verwendung einer Zange vorgesehen ist, werden die Kraftangriffsflächen der Feder für den Zangenangriff gestaltet, der formschlüssig oder kraftschlüssig sein kann.

In einer speziellen Ausführungsform der Erfindung ist die Klammer als solche Omega-förmig gestaltet, wobei dann die Kraftangriffsflächen an den nach aussen abstehenden Füssen der Schenkel bestehen. Dies führt dazu, dass man die Feder nicht zusammendrückt, sondern spreizt, um die Klammerschenkel zu öffnen. Die Füsse der Schenkel können dabei Eingriffsöffnungen für die Zangenspitzen besitzen. Andererseits lassen sich die Füsse auch in Richtung auf die Klammerschenkel zurückbiegen, so dass dann formschlüssig stumpfe, kopfartige Zangenenden zwischen die Klammerschenkel und die zurückgebogenen Füsse eingebracht werden können. Eine derartige Ausführungsform ist in besonderem Masse geeignet, um Verletzungen zu vermeiden.

Abweichend von der vorstehend beschriebenen Ausführungsform lässt sich die Klammer grundsätzlich auch U-förmig gestalten und mittels der Zangenenden spreizen. Bei dieser stark vereinfachten Ausführung kann man vor allem auch auf der Innenseite der Klammerschenkel verteilte Zähne vorsehen; desgleichen lässt sich auch nur ein Klammerschenkel endseitig als Spitze ausbilden, während der gegenüberliegende Klammerschenkel über seine Länge die erwähnten Zähne aufweist.

Eine besondere gegen eine Verkantung der Feder geschützte Ausführungsform besteht darin, dass sich zwischen der Feder und den Klammerschenkeln Verbindungsabschnitte befinden, die einander durchdringen. Zu diesem Zweck ist einer der Verbindungsabschnitte geschlitzt und der andere, gegenüberliegende Verbindungsabschnitt auf die Breite des Schlitzes reduziert, wobei dann noch beide Verbindungsabschnitte im Durchdringungsbereich entgegengesetzt zur Feder gekrümmt sind. Die Vermeidung einer Verkantung gewährleistet bei diesen sowie bei den übrigen erfindungsgemässen Messsonden das genaue Gegenüberliegen der für die Messung massgeblichen Flächen unter Einschluss der eingezogenen Hautfalte.

Bei einer anderen, grundlegenden Ausführungsform der Erfindung wird von Merkmalen der US-A-4 080 961 Gebrauch gemacht, nach denen sich an die Messsonde Ableitungskabel anschliessen, wobei die Spitzen federnde, von der Längsachse der Sonde schräg nach vorne geneigte, invasive Kontaktspitzen sind, die bei zunehmender Entspannung der auf sie einwirkenden Feder ihren Abstand voneinander bis in die Endlage verändern.

Bei dieser weiteren Ausführungsform schlägt die Erfindung zur Aufgabenlösung die Merkmale der Ansprüche 10 bis 17 vor. Danach vermögen die Kontaktspitzen unter Beibehaltung ihrer Ebene nur bis zu einer genau vorgegebenen Tiefe in die Kopfhaut einzudringen. Bei der bevorzugt mittels einer Zange vorgenommenen Applikation führt eine Entlastung der Zangendruckflächen ohne Einsatz weiterer manueller Kräfte dazu, dass die Kontaktspitzen in die Kopfhaut eindringen. Dabei besteht noch der weitere Vorteil, dass sich die Sonde mit geschlossenen Zangenspitzen einführen lässt, so dass eine minimale Ausdehnung besteht und die Anwendung auch bei nur leicht geöffnetem Muttermund möglich ist.

Die vorgeschlagenen Kunststoffumhüllungsabschnitte sind nicht nur unter dem Gesichtspunkt eines Tiefenanschlages der Kontaktspitzen zweckmässig, sondern darüber hinaus auch wegen ihrer Isolationswirkung bei der Ableitung von Potentialen sowie zur Verbesserung der Ausbildung von Zangenflächen.

Zweckmässig werden die Kontaktspitzen nach aussen gerichtet ausgeführt, wobei sie sich endseitig bei Entspannung der Feder um etwa 10 mm zu spreizen vermögen, während sie eine Länge von weniger als 3 mm aufweisen. Bei Verwendung einer Spreizfeder ist deren Kraft zweckmässig so begrenzt, dass sie sich bei massvollem Längszug der Messsonde zu schliessen sucht und letztere herauszuziehen gestattet, um zum Beispiel die Geburt durch einen Kaiserschnitt auszuführen.

Von den Kontaktspitzen geht ein erstes Ableitungskabel für das fetale EKG aus, während von einem in der Nähe der Spitze der Kunststoff-Umhüllung befindlichen stumpfen Mutterkontakt ein zweites Ableitungskabel die Ableitung des Bezugspotentials ausgeht. Beide Ableitkabel können gemeinsam auch mit zusätzlichen Kabeln eingeführt werden, die beispielsweise zu Messelementen, wie temperaturveränderlichen Widerständen, führen, welche an der mit der Haut in Berührung gelangenden Seite des Umhüllungsabschnittes vorgesehen sind. Ferner lässt sich auch wenigstens eine der Kontaktspitzen als Injektionskanüle ausführen, von der ein Schlauch ausgeht, der gemeinsam mit den Ableitungskabeln geführt sein kann.

Zur weiteren Veranschaulichung der Erfindung wird auf die sich auf Ausführungsbeispiele beziehenden Zeichnungen Bezug genommen. Darin zeigen, jeweils in schematischen Darstellungen:

Fig. 1 eine erste Ausführungsform der Erfindung, bei welcher zur besseren Darstellung der erfindungsgemässen Merkmale eine isometrische Wiedergabe gewählt wurde,

Fig. 2 und Fig. 3 weitere Ausführungsformen im Querschnitt bei Verwendung von Kreisringfedern,

Fig. 4 eine Art des Eingriffs einer Zangenspitze in einen Fuss der Messsonde,

Fig. 5 eine Omega-förmige Messsonde,

Fig. 6 eine abweichende Ausführungsform der Erfindung mit einer als Spreizfeder ausgeführten Messsonde,

Fig. 7 eine weitere Abwandlung der Messsonde mit einer Schraubenfeder, und

Fig. 8 eine Messsonde mit einer W-förmigen Feder.

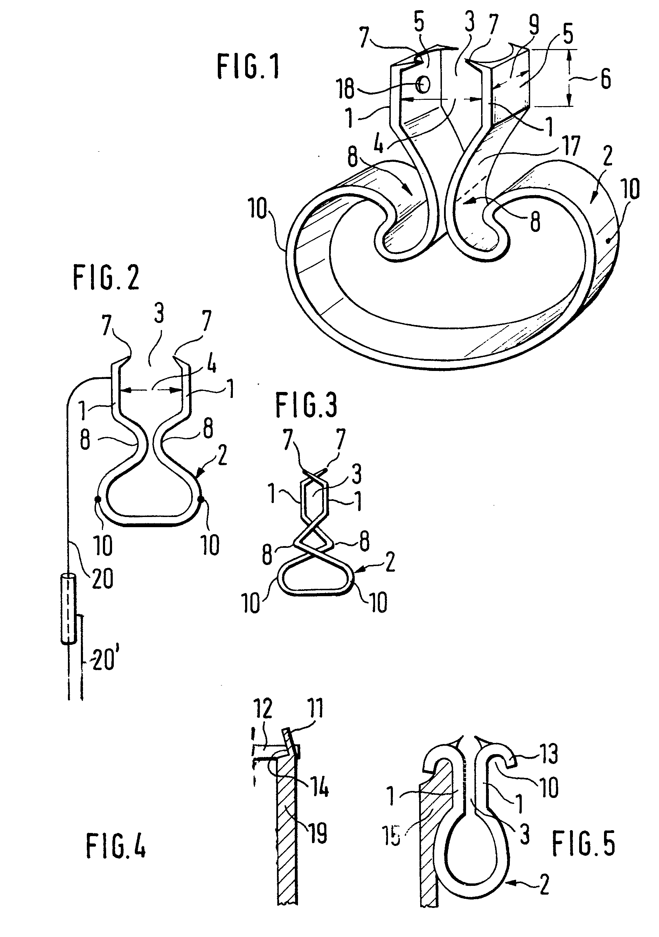

Den Ausführungsformen der Fig. 1 bis 5 sind gemeinsam die Klammerschenkel 1, welche mit der Feder 2 in Verbindung stehen, für welche die Kraftangriffsflächen 10 vorgesehen sind. Weiter ist allen Ausführungsformen der Spalt 3 zwischen den Klammerschenkeln 1 gemeinsam.

Wie vor allem Fig. 1 erkennen lässt, sind die Abmessungen des Spaltes 3 in allen Raumkoordinaten fixiert. Die Spaltweite 4 ist vom Spannungszustand der Feder abhängig. Sie wird bei gespanntem Zustand der Feder grösser, um bei Entspannung der Feder auf ein vorgebbares Mass zurückzugehen, welches der eingezogenen Hautfalte entspricht. Die Hautfalte wird auf der gesamten Breite 9 bis zur Höhe 6 in den Spalt 3 eingezogen. Die einander gegenüberliegenden Flächen der Klammerschenkel 1 sind dann im entlasteten Zustand der Feder unmittelbar an die Hautfläche angepresst, sofern auf ihnen nicht im Sinne der vorliegenden Erfindung weitere Geber von Messeinrichtungen angebracht sind, die dann an der Haut anliegen. Schematisch zeigt der linke Klammerschenkel der Fig. 1 einen aufgesetzten Beschleunigungsaufnehmer 18. Der Abstand der an den Enden der Klammerschenkel 1 vorgesehenen Zähne 7 voneinander ist stets kleiner als die Weite 4 des Spaltes 3, so dass die Hautfalte wirkungsvoll fixiert ist. Zwischen den Klammerschenkeln 1 und der Feder 2 bestehen bei den Ausführungsformen der Fig. 1 bis 3 gebogene Verbindungsabschnitte 8, deren Krümmung zu derjenigen der Feder 2 entgegengesetzt ist. Die Verbindungsabschnitte 8 berühren bei der Ausführungsform nach Fig. 1 und 2 einander, und zwar entlang einer sich in Richtung der Breite 9 des Spaltes 3 erstreckenden Linie 17. An dieser Berührungsstelle rollen die Verbindungsabschnitte 8 bei Belastung der Kraftangriffsflächen 10 aneinander ab und können damit die Klammerschenkel öffnen oder schliessen. Bei der Ausführungsform nach Fig. 3 durchdringen sich die Verbindungsabschnitte 8 in der bereits beschriebenen Weise, so dass es ebenfalls bei einer Druckbelastung der Kraftangriffsflächen 10 zu einer Öffnung des Spaltes 3 kommt.

Die Ausführungsform der Fig. 4 zeigt den Kraftangriff einer Zange 19 auf endseitig nach aussen abstehende Füsse 12 der Feder einer Klammer. Zu diesem Zweck ist die Zange 19 mit Zangenspitzen 11 versehen, die in entsprechende Eingriffsöffnungen 14 in den Füssen 12 der im übrigen hier nicht näher dargestellten Klammer einsetzbar sind.

Die Klammer ist nach Fig. 5 Omega-förmig ausgeführt. In diesem Falle sind abweichend von der Ausgestaltung nach Fig. 4 die Füsse 13 in Richtung auf die Klammerschenkel 1 zurückgebogen. Hierdurch wird Raum für den formschlüssigen, fluchtenden Eingriff eines stumpfen, kopfartigen Zangenendes 15 geschaffen. Wie die Zeichnung zeigt, lässt sich der Übergang von den Füssen zu den Zangenenden leicht so gestalten, dass eine weitergehende Abrundung besteht.

Die Ausführungsformen gemäss den Fig. 1 bis 5 besitzen Ableitkabel, wie es der Einfachheit halber nur in Fig. 2 zeichnerisch wiedergegeben ist. Das Ableitkabel 20 ist an die Innenseite eines der beiden Klammerschenkel 1 angeschlossen. Im Anschluss an die Messsonde ist das Ableitkabel 20 durch einen blanken Metallzylinder isoliert geführt, an welch letzteren sich das weitere Ableitkabel 20' isoliert anschliesst. Auf diese Weise wird ein Bezugspotential geschaffen, welches in Berührung mit der Mutter steht.

Neben dieser bei allen Ausführungsformen vorgesehenen Ableitung können, wie bereits zuvor erwähnt, ergänzende Geber weiterer Messeinrichtungen vorhanden sein, von denen Fig. 1 schematisch einen Beschleunigungsaufnehmer 18 wiedergibt. Anstelle eines derartigen Beschleunigungsaufnehmers 18 können insbesondere Elektroden für die transcutane Sauerstoffpartialdruckmessung vorhanden sein, wie sie aus der Druckschrift «Cutaneous Oxygen Monitoring in the Newborn», Paediatrician, Band 5, Nr. 5–6, 1976, Seite 342 wiedergegeben ist.

Desgleichen kann an der Innenseite eines Klammerschenkels ein Hall-Generator befestigt sein, mit dem Bewegungen relativ zum Magnetfeld der Erde erfasst werden können. Ein derartiger Hall-Generator wird unter der Typenbezeichnung SAS 231 L angeboten von der Firma Siemens Aktiengesellschaft, Erlangen, Deutschland. Man benötigt für einen derartigen Hall-Generator, der an die Stelle des Beschleunigungsaufnehmers 18 treten würde, insgesamt fünf Ableitungen, damit diese Zuleitungen für die Versorgungsspannung und die Ableitungen für die abzuleitende Spannung verwirklicht werden können. Auf eine zeichnerische Wiedergabe dieser Ableitungen wurde aus Gründen der Übersichtlichkeit verzichtet. Sie treten indes auf der freien Seite der Klammerschenkel 1 aus.

Bei einem Beschleunigungsaufnehmer würde man demgegenüber mit vier Leitungen auskommen. Eine geeignete Ausführung hat die Typenbezeichnung EGA-125-50D und wird von der Firma Entran International Fairfield, N.J., USA, geliefert.

Mit nur drei Ableitungen würde man ein herkömmliches Mikrophon anschliessen können, wohingegen für eine fotooptische Einrichtung, bestehend aus einer Fotodiode und einem Empfänger, vier Ableitungen notwendig wären.

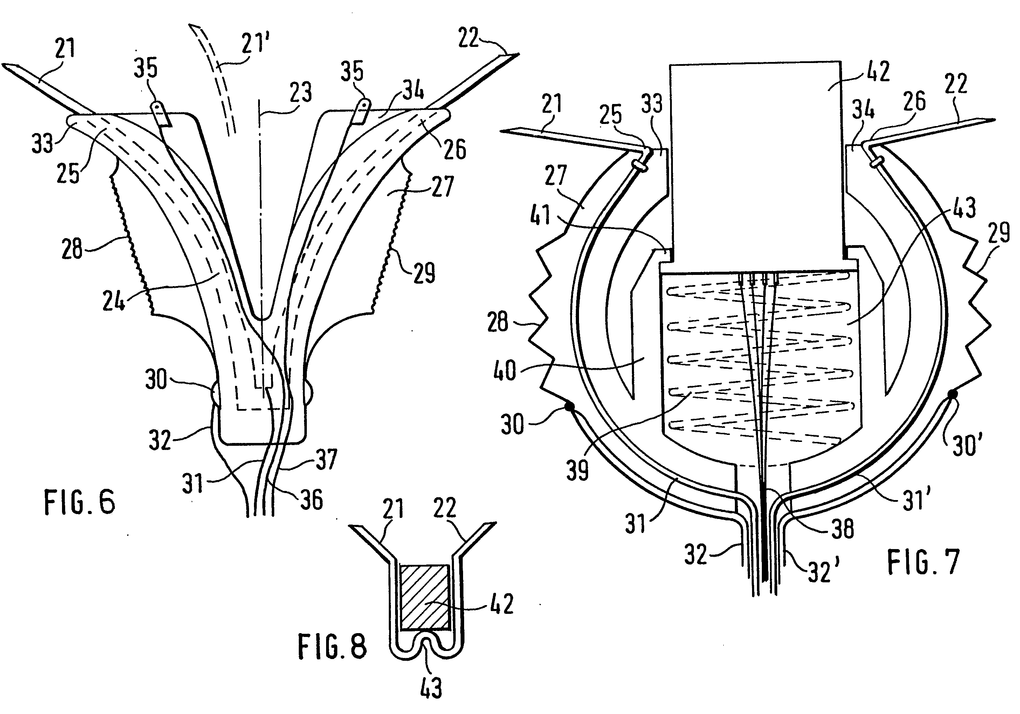

Bei der Ausführungsform nach Fig. 6 schliessen sich an die V-förmig ausgeführte Feder 24 abgewinkelte Abschnitte 25, 26 an, die zu den Kontaktspitzen 21, 22 fortgesetzt sind. Die Winkelstellung der Kontaktspitzen 21, 22 zur Längsachse 23 der Sonde ist derart, dass sie in zusammengedrücktem Zustand der V-förmigen Feder 24 eine geringere Neigung zur Längsachse 23 aufweisen als im entspannten Zustand. Die gestrichelte Linie 21' zeigt die Lage der Feder und der Kontaktspitze im gespannten Zustand. Im entspannten Zustand nehmen die Kontaktspitzen eine nahezu senkrechte Stellung in Bezug auf die Längsachse 23 ein.

Die V-förmige Kunststoffumhüllung 27 besitzt beidseitig Zangendruckflächen 28, 29, die mit Riffeln oder dergleichen griffiger gemacht werden können. Somit ist es möglich, die V-förmige Feder durch Schliessen der Zange geschlossen zu halten.

Neben den Kontaktspitzen 21, von denen ein gemeinschaftliches Ableitungskabel 31 ausgeht, ist noch eine stumpfe Elektrode 30 in Nähe der Spitze der Kunststoffumhüllung 27 vorgesehen, welche als Mutterelektrode geschaltet ist; von dieser geht das Ableitungskabel 32 aus.

Die V-förmige Umhüllung 27 umfasst auch die abgewinkelten Abschnitte 25 und 26. An dieser Stelle besitzt die Kunststoffumhüllung Abschnitte 33 und 34, welche derart flach gestaltet sind, dass sie die Eindringtiefe der Kontaktspitzen 21, 22 begrenzen. Die Abschnitte 33 und 34, tragen je ein Messelement 35, von denen eines zum Beispiel ein NTC-Widerstand ist. Von ihnen gehen je ein Ableitungskabel 36 und 37 aus. Die Kontaktspitze 22 ist zusätzlich als eine Injektionskanüle ausgeführt. An diese schliesst sich ein hier nicht dargestellter Injektionsschlauch an. Wahlweise kann sie auch den NTC-Widerstand mit einem Ableitungskabel aufnehmen.

Bei der in Fig. 7 dargestellten Ausführungsform sind die gleichen Bezugszeichen für gleiche Teile wie in Fig. 6 angegeben. Die Kunststoffumhüllung 27 ist hierbei jedoch nicht V-förmig, sondern im Querschnitt etwa kreisförmig. In ihr sind je ein Ableitkabel 31 und 31' für die beiden Kontaktspitzen 21 und 22 isoliert geführt. Ausserdem verlaufen in ihr die beiden isoliert geführten Kabel 32 und 32' des Bezugspotentials, ausgehend von den stumpfen Elektroden 30, 30'. Im Innern der Kunststoffumhüllung 27 ist ein aus Kunststoff angeformter Zylinder 40 vorgesehen, der sich in Längsrichtung der Messsonde erstreckt, und der eine Schraubendruckfeder 39 aufnimmt. Die Vorderkante des Zylinders ist mit einer ringförmigen Schulter 41 ausgeführt, an welcher in der dargestellten Lage die Gegenschulter des Gehäuses eines weiteren Messelementes 42 anliegt. In dieser Lage wird das weitere Messelement 42 von der entspannten, im Freiraum 43 befindlichen Schraubendruckfeder 39 gehalten.

Somit befindet sich die Messsonde gemäss Fig. 7 gleichfalls im Applikationszustand. Für die vorherigen Zwecke der Einführung war das Messelement 42 in den Freiraum 43 unter Spannung der Schraubendruckfeder 39 zurückgeschoben, während die Kunststoffumhüllung 27 mittels einer Zange, die an den Zangendruckflächen 28 und 29 angriff, zusammengehalten wurde. Die Entlastung der Zangendruckflächen 28 und 29 führte sodann zum Vordrücken des Messelementes 42 bei gleichzeitiger Spreizung der Kontaktspitzen 21, 22.

Das weitere Messelement 42 steht also unter der Einwirkung der Feder 39, während es in Funktion ist. Dadurch ist ein besonders wirkungsvoller Hautkontakt gewährleistet. Es kann also nicht nur ein Mikrophon sein, sondern auch etwa ein Beschleunigungsaufnehmer oder ein Sensor zur Bestimmung der Raumkoordinaten bzw. ein Sensor für Reflexions- und Transmissionsmessungen. Die Transmission ist dann möglich, wenn sich die Kontaktspitzen 21, 22 unter der Haut gegenüber liegen. Diese Messungen sind für die laufende Überwachung von grossem Interesse, weil man hierdurch auf den Grad der Durchblutung und damit der akut veränderten Kreislaufsituation, wie zum Beispiel Zentralisation, schliessen kann. Lichtquellen und Fotoempfänger lassen sich im Bereich der Kontaktspitzen hierfür anordnen.

Wie erwähnt, kann wenigstens eine der Kontaktspitzen als eine Injektionskanüle ausgeführt sein. Dies ermöglicht nicht nur den Anschluss einer Schlauchleitung, sondern man kann die Injektionskanüle auch benutzen, um eine pH-Messelektrode einzuführen. Eine derartige Messelektrode ist bekannt aus der Druckschrift «Innovation in Instrumentation» der Firma World Prezision Instruments, Inc., New Haven Conn., USA, und dort als Beetrode näher bezeichnet. Eine derartige pH-Messelektrode kann in einer als Injektionskanüle ausgeführten Kontaktspitze weitgehend geführt und gegen Abbrechen gesichert werden.

Fig. 8 zeigt eine Anordnung, bei welcher sich die Kontaktspitzen 21 und 22 an den Enden einer etwa W-förmigen Feder befinden. Diese Darstellung ist im Zustand der Spannung wiedergegeben. Im Zustand der Entspannung spreizen sich die Kontaktspitzen 21 und 22 auseinander und nehmen eine durch den Entspannungszustand der Feder vorgegebene, fixierte Stellung in der Kopfhaut ein. Dabei wird der Innenabschnitt 43 der Feder in Richtung auf die Kopfhaut gedrückt und presst dabei das Messelement 42 an die Kopfhaut an. In diesem Falle ist es auch möglich, anstelle des Messelementes einen Bolzen zu verwenden, der nur die Funktion übernimmt, einen zuverlässigen Anpressungszustand herbeizuführen.

**Patentansprüche**

1. Physiologische Messsonde, insbesondere transzervikal mit Spitzen (7, 7') an der fetalen Kopfhaut fixierbare Vaginalelektrode für die Ableitung des fetalen EKG, an die sich ein Ableitkabel (20) anschliesst, wobei die Sonde als Klammer ausgebildet ist, deren Klammerschenkel mit den nach innen gerichteten Spitzen versehen sind, deren Abstand voneinander kleiner als der Abstand der Klammerschenkel (1) ist, und an die sich eine Feder (2) anschliesst, deren gespanntem Zustand die Öffnungslage der Klammerschenkel (1) entspricht, und die sich bei Entlastung der Feder (2) in Richtung aufeinander bewegen, dadurch gekennzeichnet, dass sich die aus flachem Band bestehenden Klammerschenkel (1) in gerader Richtung erstrecken, wobei ihre einander zugekehrten Flächen (5) etwa parallel verlaufen und die Höhe (6) und die Breite (9) eines Spaltes (3) für die eingezogene Kopfhautfalte begrenzen, während die Spaltweite (4) im entlasteten Zustand der Feder (2) einen vorgebbaren Wert annimmt.

2. Physiologische Messsonde nach Anspruch 1, dadurch gekennzeichnet, dass die Feder (2) im wesentlichen kreisringförmig gebogen ist (Fig. 1 bis 5).

3. Physiologische Messsonde nach Anspruch 2, dadurch gekennzeichnet, dass zwischen der Feder (2) und den Klammerschenkeln (1) entgegengesetzt zur Federrichtung gebogene Verbindungsabschnitte (8) vorgesehen sind, die sich entlang einer sich in Richtung der Breite (9) des Spaltes (3) erstreckenden Linie (17) in jedem Spannungszustand der Feder (2) berühren.

4. Physiologische Messsonde nach einem oder mehreren der Ansprüche 1 bis 3, dadurch gekennzeichnet, dass die Feder (2) mit Kraftangriffsflächen (10) für die Anlage einer Betätigungszange ausgebildet ist.

5. Physiologische Messsonde nach Anspruch 4, dadurch gekennzeichnet, dass die Klammer Omega-förmig (Fig. 4 und 5) ausgeführt ist, wobei die Kraftangriffsflächen (10) an den nach aussen abstehenden Füssen (12, 13) der Klammerschenkel (1) bestehen.

6. Physiologische Messsonde nach Anspruch 5, dadurch gekennzeichnet, dass die Füsse (13) der Klammerschenkel (1) für den fluchtenden und formschlüssigen Eingriff von stumpfen, kopfartigen Zangenenden (15) parallel zu den Klammerschenkeln (1) zurückgebogen sind.

7. Physiologische Messsonde nach Anspruch 4, dadurch gekennzeichnet, dass die Klammer U-förmig gestaltet und mittels Zangenenden spreizbar ist.

8. Physiologische Messsonde nach den Ansprüchen 1 und 2, dadurch gekennzeichnet, dass sich Verbindungsabschnitte (8) zwischen der Feder (2) und den Klammerschenkeln (1) einander durchdringen, indem der Durchdringungsbereich bei dem einen Verbindungsabschnitt geschlitzt und bei dem anderen Verbindungsabschnitt dem Schlitz gegenüberliegend bis auf dessen Breite reduziert ist, und beide Verbindungsabschnitte im Durchdringungsbereich entgegengesetzt zur Feder (2) gekrümmt sind.

9. Physiologische Messsonde nach einem oder mehreren der Ansprüche 1 bis 8, dadurch gekennzeichnet, dass die Innenseiten der Klammerschenkel (1) für die Aufnahme von Gebern weiterer Messeinrichtungen ausgeführt sind.

10. Physiologische Messsonde, insbesondere transzervikal mittels Spitzen an der fetalen Kopfhaut fixierbare Vaginalelektrode für die Ableitung des fetalen EKG, an die sich Ableitungskabel anschliessen, wobei die Spitzen federnde, von der Längsachse (23) der Sonde schräg nach vorne geneigte, invasive Kontaktspitzen sind, die bei zunehmender Entspannung der auf sie einwirkenden Feder (24, 39) ihren Abstand voneinander bis in die Endlage verändern, dadurch gekennzeichnet, dass die Kontaktspitzen (21, 22) ihren Abstand voneinander unter Beibehaltung der von ihnen gebildeten Ebene bei zunehmender Entspannung der auf sie einwirkenden Feder (24, 39) verändern, wobei die Endlage der Kontaktspitzen (21, 22) innerhalb der von ihnen gebildeten Ebene durch die Feder (24, 39) in deren entlastetem Zustand fixiert ist, und die Eindringtiefe der Kontaktspitzen (21, 22) durch als Tiefenanschlag ausgebildete Kunststoffumhüllungsabschnitte (33, 34) begrenzt ist.

11. Physiologische Messsonde nach Anspruch 10, dadurch gekennzeichnet, dass die Kontaktspitzen (21, 22) eine definierte Länge von weniger als 3 mm aufweisen und voneinander weg nach aussen gerichtet sind, wobei sie sich aus ihrer Schliesslage bei Entspannung der Feder (24, 39) um etwa 10 mm zu spreizen vermögen.

12. Physiologische Messsonde nach einem oder beiden der Ansprüche 10 und 11, dadurch gekennzeichnet, dass die Feder (24) beidendig abgewinkelte Abschnitte (25, 26) mit den sich daran anschliessenden Kontaktspitzen (21, 22) aufweist, wobei die Feder (24) einschliesslich der Abschnitte eine isolierende Kunststoffumhüllung (27, 33, 34) aufweist.

13. Physiologische Messsonde nach einem oder mehreren der Ansprüche 10 bis 12, dadurch gekennzeichnet, dass mit dem Ableitkabel (31) ein zweites Ableitkabel (32) für die Ableitung eines Bezugspotentials gemeinsam einführbar ist, das von den Kontaktspitzen (21, 22) beabstandet endigt.

14. Physiologische Messsonde nach einem oder mehreren der Ansprüche 10 bis 13, dadurch gekennzeichnet, dass an jede der Kontaktspitzen (21, 22) ein eigenes Ableitkabel (31, 31') angeschlossen ist.

15. Physiologische Messsonde nach einem oder mehreren der Ansprüche 10 bis 14, dadurch gekennzeichnet, dass eine der Kontaktspitzen (22) als Injektionskanüle ausgeführt ist, von der eine Schlauchleitung ausgeht.

16. Physiologische Messsonde nach einem oder mehreren der Ansprüche 10 bis 15, dadurch gekennzeichnet, dass eine der Kontaktspitzen (22) ein weiteres Messelement, insbesondere einen temperaturabhängigen Widerstand trägt, und dass dafür eine Kabelableitung besteht.

17. Physiologische Messsonde nach einem oder mehreren der Ansprüche 10 bis 16, dadurch gekennzeichnet, dass zwischen den Kontaktspitzen (21, 22) ein ein weiteres Messelement (42), insbesondere ein Mikrophon, aufnehmender Freiraum (43) besteht, wobei das Messelement (42) in Richtung der Längsachse der Sonde von der Feder (39) derart belastet ist, dass es bei Entspannung der Feder (39) nach vorne gedrückt ist.

**Claims**

1. Physiological measuring probe, especially transcervical, with tips (7, 7') at the vaginal electrode fixable to the fetal head skin for the lead of the fetal electrocardiogram, with a connected lead cable (20), the probe being designed in form of a clamp, the clamping legs of which are provided with the inward directed tips, the spacing of which is smaller than the spacing of the clamping legs (1), and to which a spring (2) is connected, the tensioned state of said spring corresponding to the open position of the clamping legs (1), and which move towards one another when the spring is being relaxed, characterized in that the clamping legs (1) consisting of flat strip extend in straight direction, the mutually facing surfaces (5) of which are approximately parallel and limit the height (6) and the width (9) of a gap (3) for the drawn-in wrinkle of the head skin, whereas the gap width (4) assumes a preselectable value in the relaxed state of the spring (2).

2. Physiological measuring probe according to claim 1, characterized in that the spring (2) is basically bent to an annular shape (Fig. 1 to 5).

3. Physiological measuring probe according to claim 2, characterized in that curved connecting sections (8) are provided between the spring (2) and the clamping legs (1) in opposition to the direction of the spring, which are in contact along a line (17) extending in the direction of the width (9) of the gap every time the spring (2) is in a tensioned state.

4. Physiological measuring probe according to one or several of the claims 1 to 3, characterized in that the spring (2) is designed with surfaces of application of force (10) for the employment of a pair of actuating tongs.

5. Physiological measuring probe according to claim 4, characterized in that the clamp is omega-shaped (Fig. 4 and 5), the surfaces of application of force (10) being formed by the outward projecting feet (12, 13) of the clamping legs (1).

6. Physiological measuring probe according to claim 5, characterized in that the feet (13) of the clamping legs (1) are bent backwards parallel to the clamping legs (1) for the flush and positive action of blunt, head-type tong-holds (15).

7. Physiological measuring probe according to claim 4, characterized in that the clamp is U-shaped and expendable by means of tong-holds.

8. Physiological measuring probe according to claims 1 and 2, characterized in that the connecting sections (8) arranged between the spring (2) and the clamping legs (1) intersect, the intersection area being slotted in one of the connecting sections and reduced opposite the slot to the width of same in the other connecting section, and that both connecting sections are bent in the intersection area in opposition to the spring (2).

9. Physiological measuring probe according to one or several of the claims 1 to 8, characterized in that the inner sides of the clamping legs (1) are designed to accommodate the transducers of additional measuring devices.

10. Physiological measuring probe, especially transcervical by means of tips of the vaginal electrode fixable to the fetal head skin for the lead of the fetal electrocardiogram, with connected lead cables, the tips being spring-mounted, invasive contact tips bent forward from the longitudinal axis (23) of the probe, which change their spacing to one another up to the limit position at increasing relaxing of the springs (24, 39) acting on the tips, characterized in that the contact tips (21, 22) change their spacing to one another while retaining the planes formed by same at increasing relaxing of the springs (24, 39) acting on the tips, the limit position of the contact tips (21, 22) being fixed within the plane formed by same by the springs (24, 39) in their relaxed state, and the depth of penetration of the contact tips (21, 22) being limited by the plastic sheathing sections (33, 34) designed as depth stop.

11. Physiological measuring probe according to claim 10, characterized in that the contact tips (21, 22) have a definite length of less than 3 mm and are directed outward away from one another, said contact tips (21, 22) being capable of spreading apart by about 10 mm from their closed position upon relaxing of the springs (24, 39).

12. Physiological measuring probe according to one or both claims 10 and 11, characterized in that the spring (24) features at both ends angular sections (25, 26) with the subsequent contact tips (21, 22), the spring (24) inclusive of the sections featuring an insulating plastic sheathing (27, 33, 34).

13. Physiological measuring probe according to one or several of the claims 10 to 12, characterized in that a second lead cable (32) is jointly insertable with the lead cable (31) for the derivation of a reference potential, said lead cable (32) terminating at a distance from the contact tips (21, 22).

14. Physiological measuring probe according to one or several of the claims 10 to 13, characterized in that a separate lead cable (31, 31') is connected to each of the contact tips (21, 22).

15. Physiological measuring probe according to one or several of the claims 10 to 14, characterized in that one of the contact tips (22) is designed as injection cannula with an outgoing hose pipe.

16. Physiological measuring probe according to one or several of the claims 10 to 15, characterized in that one of the contact tips (22) carries an additional measuring element, especially a thermistor, and that a cable lead is provided therefor.

17. Physiological measuring probe according to one or several of the claims 10 to 16, characterized in that between the contact tips (21, 22) there exists a clearance (43) for the accommodation of an additional measuring element (43), especially a microphone, the measuring element (43) being loaded by the spring (39) in the direction of the longitudinal axis of the probe in such a way that it is pressed forward when the spring (39) is relaxed.

## Revendications

1. Sonde de mesure physiologique, en particulier avec électrodes vaginales fixables transcervicalement par des pointes (7, 7') sur la peau de la tête du fœtus pour la transmission de l'électro-encéphalogramme du fœtus, auquel est rattaché un câble de transmission (20), la sonde étant conçue en tant que pince dont les branches sont équipées de pointes situées vers l'intérieur et dont l'écartement de l'une par rapport à l'autre est inférieur à l'écartement des branches de la pince (1), sur lesquelles un ressort (2) dont la situation tendue correspond à la situation d'ouverture des branches de la pince (1) qui se rapprochent l'une de l'autre lors du relâchement du ressort (2), caractérisée en ce que les branches de la pinces (1) constituées de fer plat s'étendent en direction rectiligne, leur surfaces opposées l'une à l'autre (5) étant pratiquement parallèles et limitant la hauteur (6) et la largeur (9) d'une fente (3) du pli de la peau de la tête tiré, cependant que la largeur de la fente (4) prend une valeur réglable dans la situation relâchée du ressort.

2. Sonde de mesure physiologique selon la revendication 1, caractérisée en ce que le ressort (2) est recourbé en prépondérance en forme d'anneau (Fig. 1 à 5).

3. Sonde de mesure physiologique selon la revendication 2, caractérisée en ce que des sections de liaison (8) recourbées dans le sens inverse de celui du ressort et qui se touchent alors de chaque tension du ressort (2) le long d'une ligne (17) s'écoulant en direction de la largeur (9) de la fente (3) sont prévues.

4. Sonde de mesure physiologique selon l'une ou plusieurs des revendications 1 à 3, caractérisée en ce que le ressort (2) est formée avec des surfaces d'attaque de force (10) pour la mise en place d'une pince de manœuvre.

5. Sonde de mesure physiologique selon la revendication 4, caractérisée en ce que la pince est conçue en forme d'Oméga (fig. 4 et 5), les surfaces d'attaque de force (10) existent sur les pieds (12, 13) dépassant vers l'extérieur des branches de la pince (11).

6. Sonde de mesure physiologique selon la revendication 5, caractérisée en ce que les pieds (13) de la branche de la pince (1) sont repliés en arrière parallèlement aux branches de la pince (1) pour la prise alignée et adaptée du point de vue de la forme d'extrémités de pinces (15) arrondies et semblables à une tête.

7. Sonde de mesure physiologique selon la revendication 4, caractérisée en ce que la pince est conçue en forme de U et est ouvrable au moyen d'extrémités de pinces.

8. Sonde de mesure physiologique selon les revendications 1 et 2, caractérisée en ce que les sections de raccordement (8) entre le ressort (2) et les branches de la pince (1) s'interpénètrent, le secteur d'interpénétration étant fendu d'un côté et réduit, du côté opposé à la fente jusqu'à la largeur de ladite, les deux sections de raccordement étant courbées, dans le secteur d'interpénétration, à l'inverse du ressort (2).

9. Sonde de mesure physiologique selon l'une ou plusieurs des revendications 1 à 8, caractérisée en ce que les côtés intérieurs des branches de la pince (1) sont exécutées pour la réception de capteurs d'équipements de mesure supplémentaires.

10. Sonde de mesure physiologique, en particulier avec électrodes vaginales fixables transcervicalement par des pointes sur la peau de la tête du fœtus pour la transmission de l'électro-encéphalogramme du fœtus, auquel est rattaché un câble de transmission, les pointes étant des pointes de contact invasives élastiques orientées obliquement vers l'avant par rapport à l'axe longitudinal (23) de la sonde, qui modifient, au fur et à mesure du relâchement des ressorts agissant sur elles (24, 39) leur écartement l'une de l'autre jusqu'en leur position finale, caractérisée en ce que les pointes de contact (21, 22) modifient leur écartement l'une de l'autre lors du relâchement des ressorts (24, 39) agissant sur elles en conservant le plan qu'elles forment, la situation finale des pointes de contact (21, 22) étant fixée au sein du plan qu'elles forment par les ressorts (24, 39) détendus, et la profondeur de pénétration des pointes de contact (21, 22) étant limitée par des sections à enrobage en matière plastique (33, 34) servant de butée de profondeur.

11. Sonde de mesure physiologique selon la revendication 10, caractérisée en ce que les pointes de contact (21, 22) présentent une longueur définie de moins de 3 mm et sont orientées en s'écartant l'une de l'autre vers l'extérieur, et peuvent, en situation de repos des ressorts (24, 39), s'écarter d'environ 10 mm de leur position finale.

12. Sonde de mesure physiologique selon l'une des revendications 10 et 11, caractérisée en ce que le ressort (24) présente à ses deux extrémités des sections courbées (25, 26) avec les pointes de contact y faisant suite, le ressort (24) y compris ces sections présentant un enrobage en matière plastique (27, 33, 34).

13. Sonde de mesure physiologique selon l'une ou plusieurs des revendications 10 à 12, caractérisée en ce qu'un deuxième câble de transmission (32) s'achevant loin des pointes de contact (21, 22) peut être introduit en même temps que le câble de transmission (31) pour la transmission d'un potentiel de référence.

14. Sonde de mesure physiologique selon l'une ou plusieurs des revendications 10 à 13, caractérisée en ce qu'un câble de transmission (31, 31') propre à chaque pointe de contact (21, 22) est raccordé à chacune de ces pointes.

15. Sonde de mesure physiologique selon l'une ou plusieurs des revendications 10 à 14, caractérisée en ce que l'une des pointes de contact (22) est exécutée en tant que canule d'injection partant d'une canalisation à flexible.

16. Sonde de mesure physiologique selon l'une ou plusieurs des revendications 10 à 15, caractérisée en ce que l'une des pointes de contact (22) porte un élément de mesure supplémentaire, en particulier une résistance dépendant de la température, et qu'il existe, pour celui-ci, une dérivation de câble.

17. Sonde de mesure physiologique selon l'une ou plusieurs des revendications 10 à 16, caractérisée en ce que il existe, entre les pointes de contact (21, 22), un espace libre (43) acceptant un élément de mesure supplémentaire (42), en particulier un microphone, l'élément de mesure (42) étant sollicité par le ressort (39) dans le sens de l'axe longitudinal de sorte telle qu'il est poussé vers l'avant lors.

FIG.1

FIG.2

FIG.3

FIG.4

FIG.5

FIG.6

FIG.8

FIG.7

0 137 500